**Europäisches Patentamt**

**European Patent Office**

**Office européen des brevets**

(19)

(11) Veröffentlichungsnummer: **0 205 878**
**B1**

(12) **EUROPÄISCHE PATENTSCHRIFT**

(45) Veröffentlichungstag der Patentschrift:
**26.04.89**

(51) Int. Cl.⁴: **A61B 17/22**

(21) Anmeldenummer: **86106485.5**

(22) Anmeldetag: **13.05.86**

(54) **Einrichtung zum berührungslosen Zertrümmern von Konkrementen.**

(30) Priorität: **28.05.85 DE 3519127**

(43) Veröffentlichungstag der Anmeldung:
**30.12.86 Patentblatt 86/52**

(45) Bekanntmachung des Hinweises auf die Patenterteilung:
**26.04.89 Patentblatt 89/17**

(84) Benannte Vertragsstaaten:
**DE GB**

(56) Entgegenhaltungen:
**EP-A- 0 081 639**
**DE-A- 2 722 252**
**DE-A- 2 852 560**
**DE-A- 3 122 056**
**DE-A- 3 220 751**
**DE-A- 3 336 252**

(73) Patentinhaber: **Siemens Aktiengesellschaft Berlin und München, Wittelsbacherplatz 2, D-8000 München 2(DE)**

(72) Erfinder: **Hahn, Alfred, Bunsenstrasse 64, D-8520 Erlangen(DE)**
Erfinder: **Vogel, Georg, Am Eichengarten 9, D-8520 Buckenhof(DE)**

ACTORUM AG

**Beschreibung**

Die Erfindung betrifft eine Einrichtung zum berührungslosen Zertrümmern eines im Körper eines Lebewesens befindlichen Konkrementes mit einem Stoßwellengenerator, der auf ein Zielgebiet im Körper ausrichtbar ist, bei der der Stoßwellengenerator über eine von einem Koppelmedium gefüllte Blase an das Lebewesen ankoppelbar ist.

Einrichtungen dieser Art werden in der Medizin eingesetzt, z.B. zum Zerstören von Nierensteinen. Sie sind besonders vorteilhaft, da sie jeglichen Eingriff in den Körper vermeiden.

In der DE-OS 3 328 051 ist eine Einrichtung dieser Art beschrieben, bei der der Stoßwellengenerator als Stoßwellenrohr ausgebildet ist, dessen eines Ende durch eine mit einem Koppelmedium gefüllte Blase abgeschlossen ist. Mit diesem Ende wird der Stoßwellengenerator an das Lebewesen angelegt. Die Stoßwellen werden dabei auf einen Fokuspunkt im Zielgebiet fokussiert. Es ist daher erforderlich, das Lebewesen in bezug auf den Stoßwellengenerator so zu positionieren, daß das zu zertrümmernde Konkrement genau im Zielgebiet, im sogenannten Isozentrum, liegt.

Der Erfindung liegt die Aufgabe zugrunde, eine Einrichtung der eingangs genannten Art so auszubilden, daß eine einfache Ortung der zu behandelnden Konkremente und Zentrierung des Stoßwellengenerators möglich ist, wobei der Stoßwellengenerator so ausrichtbar ist, daß die Stoßwellen empfindliche Körperorgane nach Möglichkeit nicht treffen.

Diese Aufgabe ist erfindungsgemäß dadurch gelöst, daß der Stoßwellengenerator an einem auf dem Boden verfahrbaren Wagen mit nach oben gerichteter Blase um eine horizontale Achse schwenkbar befestigt ist, welche parallel zur Längsachse einer Patientenliege über dieser durch das Isozentrum verläuft und daß eine Röntgeneinheit vorhanden ist, bei der Röntgenstrahler und Strahlenempfänger an den Enden eines C-förmigen Trägers befestigt sind, welche die Patientenliege seitlich umgreift und um die gleiche Achse wie der Stoßwellengenerator schwenkbar ist. Bei der erfindungsgemäßen Einrichtung ist bei aus dem zu behandelnden Gebiet herausgefahrenen Stoßwellengenerator eine Ortung und Ausrichtung des Konkrementes mit Hilfe der Röntgeneinheit möglich. Danach wird der Stoßwellengenerator in seine Arbeitsposition gefahren, in der die Stoßwellen auf das Isozentrum fokussiert sind.

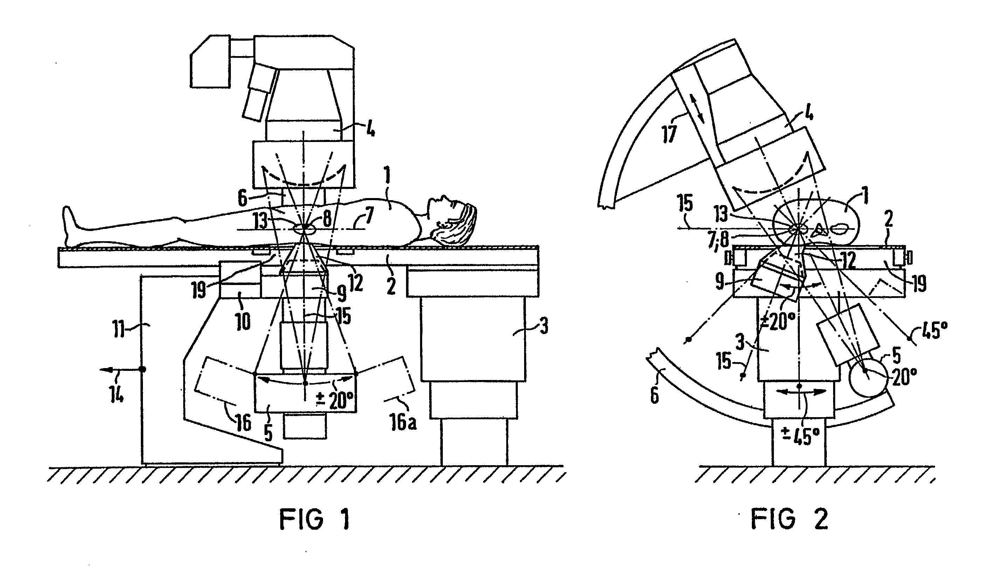

Die Erfindung ist nachfolgend anhand eines in den Figuren 1 und 2 in zwei verschiedenen Ansichten dargestellten Ausführungsbeispieles näher erläutert.

In den Figuren ist ein Patient 1 dargestellt, der auf einer Patientenliege 2 liegt, welche an einem Sockel 3 höhenverstellbar befestigt ist. Die Patientenliege 2 ist in bezug auf den Sockel 3 auch in ihrer Längs- und Querrichtung verstellbar.

Zur Ortung und Ausrichtung des Patienten 1 ist eine Röntgeneinheit vorhanden, die einen Röntgenbildverstärker 4 und einen Röntgenstrahler 5 aufweist. Die Komponenten 4, 5 sind an den Enden eines C-förmigen Trägers 6 befestigt, welcher die Patientenliege 2 seitlich umgreift und um eine horizontale Achse 7 schwenkbar ist, welche parallel oberhalb der Patientenliege 2 in deren Längsrichtung verläuft und durch das Isozentrum 8 geht.

Die Halterung für den Träger 6 (in Fig. 1, 2 nicht dargestellt) kann um eine Querachse 15 drehbar angeordnet werden, wodurch zusätzlich eine Schrägeinstrahlung in Patientenrichtung ermöglicht wird. Dies ist in Figur 1 mit ±20°-Endstellungen 16, 16a angedeutet. Die Querachse 15 verläuft dabei senkrecht zur Achse 7 in einer zur Patientenliege 2 parallelen Ebene.

Der Bildverstärker 4 kann in seiner Lage zum Isozentrum 8 an die Patientendicke über eine Führung 17 angepaßt werden.

Auf das Isozentrum 8 ist ein Stoßwellengenerator 9 in seiner Arbeitsstellung ausgerichtet. Der Stoßwellengenerator 9 ist an einem C-förmigen Träger 10 befestigt, welcher seinerseits mit einem auf dem Boden in Richtung der Achse 7 verfahrbaren Wagen 11 verbunden ist. Der Stoßwellengenerator 9 weist an seiner Oberseite eine Blase 12 auf, die mit einem Koppelmedium, z.B. mit Wasser, aufpumpbar ist.

Zur Ankopplung des Stoßwellengenerators 9 ist die Patientenliege 2 im Nierenbereich von einem Ausschnitt 19 durchbrochen.

Zur Zertrümmerung eines Konkrements, z.B. eines Nierensteines 13, wird der Stoßwellengenerator 9 zunächst bei druckloser Blase 12 in Richtung des Pfeiles 14 aus dem Behandlungsgebiet herausgefahren und der Patient 1 so lange in Längs- und Querrichtung und in seiner Höhe verstellt, bis der Nierenstein 13 im Isozentrum 8 liegt. Die Ausrichtung des Patienten 1 erfolgt mit Hilfe des durch den Röntgenbildverstärker 4 erzeugten Röntgenbildes. Dabei ist eine Durchstrahlung des Patienten 1 unter verschiedenen Richtungen durch Drehung der Röntgeneinheit 4, 5, 6 um die Achsen 7 und 15 möglich.

Nach dieser Ausrichtung wird der Wagen 11 entgegen dem Pfeil 14 so weit verschoben, bis der Stoßwellengenerator 9 in seiner Arbeitsstellung unter dem Isozentrum 8 liegt. Anschließend wird die Blase 12 mit dem Koppelmedium aufgepumpt, bis sie an der Körperoberfläche des Patienten 1 liegt. Dann kann die Stoßwellenbehandlung erfolgen.

Die Figur 2 zeigt, daß der Stoßwellengenerator 9 am in Figur 2 nicht dargestellten Träger 10 derart gelagert ist, daß seine Achse 15 gegenüber der Vertikalen um ±20° schwenkbar ist. Dadurch ist es möglich, die Einstrahlrichtung optimal zu wählen, so daß diejenigen Körperorgane, die nicht von Stoßwellen getroffen werden sollen, geschont werden können. Ferner ist die Röntgeneinheit 4, 5, 6 bei der Behandlung durch Schwenken des C-Trägers 6 so in bezug auf den Stoßwellengenerator 9 einstellbar, daß dieser im Röntgenbild nicht abgebildet wird oder zumindest nicht stört.

Auch eine kardanische Aufhängung um das Isozentrum 8 kann realisiert werden, die zusätzlich eine Schrägeinstrahlung in Patientenlängsrichtung erlaubt.

Zusammenfassend wird hinsichtlich der dargestellten Einrichtung folgendes festgehalten:

1. Es ist eine Ortung und Einstellung des zu behandelnden Konkrementes möglich, bevor der Stoßwellengenerator in seine Arbeitsstellung gefahren wird.

2. Die Ortung kann unter verschiedenen Richtungen erfolgen, da der C-Träger 6 um die Achse 7 und um eine senkrecht dazu in einer zur Patientenliege 2 parallelen Ebene liegende Achse 15 schwenkbar ist.

3. Der Röntgenbildverstärker 4 kann in seiner Größe den jeweiligen Erfordernissen angepaßt werden.

4. Es sind Röntgenbilder mit unterhalb und oberhalb der Patientenliege 2 liegendem Röntgenstrahler 5 möglich. Bei oberhalb der Patientenliege 2 liegendem Röntgenstrahler 5 ergeben sich gute geometrische Aufnahmebedingungen für die Abschlußaufnahme in a.p.-Richtung. Bei unterhalb der Patientenliege 2 liegendem Röntgenstrahler 5 ergibt sich ein guter Strahlenschutz.

5. Der um die Achse 7 kontinuierlich um ±20° schwenkbare Stoßwellengenerator 5 erlaubt eine optimale Einstrahlung auf das Isozentrum 8.

6. Die Einrichtung ist universell anwendbar, z.B. ist auch eine perkutane Steinentfernung bei wechselbarer Patientenliege möglich.

**Patentansprüche**

1. Einrichtung zum berührungslosen Zertrümmern eines im Körper eines Lebewesens (1) befindlichen Konkrementes (13) mit einem Stoßwellengenerator (9), der auf ein Zielgebiet (8) im Körper ausrichtbar ist, bei der der Stoßwellengenerator (9) über eine von einem Koppelmedium gefüllte Blase (12) an das Lebewesen (1) ankoppelbar ist, dadurch gekennzeichnet, daß der Stoßwellengenerator (5) an einem auf dem Boden verfahrbaren Wagen (11) mit nach oben gerichteter Blase (12) um eine horizontale Achse (7) schwenkbar befestigt ist, welche parallel zur Längsachse einer Patientenliege (2) oberhalb dieser durch das Zielgebiet (8) verläuft, und daß eine Röntgeneinheit (4, 5, 6) vorhanden ist, bei der Röntgenstrahler (5) und Strahlenempfänger (4) an den Enden eines C-förmigen Trägers (6) befestigt sind, welche die Patientenliege (2) seitlich umgreift und um die gleiche Achse (7) wie der Stoßwellengenerator (9) schwenkbar ist.

2. Einrichtung nach Anspruch 1, dadurch gekennzeichnet, daß die Röntgeneinheit (4, 5, 6) außer um die Achse (7) noch um eine senkrecht dazu in einer zur Patientenliege (2) parallelen Ebene verlaufende Achse (15) schwenkbar ist.

**Claims**

1. An apparatus for remote shattering of a calculus (13) present in the body of a living organism (1), having a shock-wave generator (9) which can be directed at a target area (8) in the body, wherein the shock-wave generator (9) can be coupled to the living organism (1) by means of a bladder (12) filled with a coupling medium, characterised in that the shock-wave generator (5) is mounted on a carriage (11), which can be moved along the floor, with its bladder (12) pointing upwards, so as to be pivotable about a horizontal axis (7) which extends parallel to and above the longitudinal axis of the patient couch (2) through the target area (8) and in that there is an X-ray unit (4, 5, 6) the X-ray tube (5) and radiation receiver (4) being secured to the ends of a C-shaped carrier (6) which embraces the patient couch (2) laterally and which is pivotable about the same axis (7) as the shock-wave generator (9).

2. An apparatus according to claim 1, characterised in that the X-ray unit (4, 5, 6) is not only pivotable about the axis (7) but also about an axis (15) perpendicular thereto extending in a plane parallel to the patient couch (2).

**Revendications**

1. Dispositif pour détruire à distance une concrétion (13) située dans le corps d'un être vivant (1), comportant un générateur d'ondes de choc (9), qui peut être orienté sur une région-cible (8) dans le corps, le générateur d'ondes de choc (9) pouvant être accouplé à l'être vivant (1) par l'intermédiaire d'une enceinte (12) remplie par un milieu de couplage, caractérisé par le fait que le générateur d'ondes de choc (5) est fixé sur un chariot (11) déplaçable sur le sol et équipé de l'enceinte (12) dirigée vers le haut, de manière à pouvoir tourner autour d'un axe horizontal (7) qui est parallèle à l'axe longitudinal d'une couchette pour patient (2) et s'étend au-dessus de cette dernière à travers la région-cible (8), et qu'il est prévu une unité radiologique (4, 5, 6), dans laquelle un émetteur radiologique (5) et un récepteur de rayonnement (4) sont fixés aux extrémités d'un bras (6) en forme de C, qui entoure latéralement la couchette pour patient (2) et peut pivoter autour du même axe (7) que le générateur d'ondes de choc (9).

2. Dispositif suivant la revendication 1, caractérisé par le fait que l'unité radiologique (4, 5, 6) peut pivoter non seulement autour de l'axe (7), mais également autour d'un axe (15) perpendiculaire à l'autre axe dans un plan parallèle à la couchette pour patient (2).

FIG 1

FIG 2

EP 0 205 878 B1